(19) Europäisches Patentamt
European Patent Office
Office européen des brevets

(11) **EP 0 697 596 B1**

(12) **EUROPÄISCHE PATENTSCHRIFT**

(45) Veröffentlichungstag und Bekanntmachung des Hinweises auf die Patenterteilung:
**24.05.2000 Patentblatt 2000/21**

(51) Int Cl.⁷: **G01N 33/68**, G01N 33/574, G01N 33/543, G01N 33/96

(21) Anmeldenummer: **95112891.7**

(22) Anmeldetag: **16.08.1995**

(54) **Verfahren zur immunologischen Bestimmung von Proteinen, Kit zur Durchführung des Verfahrens und für ein solches Verfahren geeignete Teströhrchen**

Method for the immunological determination of proteins, kit to carry out this method and suitable test tubes for this method

Procédé de détermination immunologique de protéines, garniture de la mise en oeuvre du procédé et éprouvettes appropriées au tel procédé

(84) Benannte Vertragsstaaten:
**AT BE CH DE DK ES FR GB IT LI NL SE**

(30) Priorität: **19.08.1994 DE 4429409**
**20.02.1995 DE 19505732**

(43) Veröffentlichungstag der Anmeldung:
**21.02.1996 Patentblatt 1996/08**

(73) Patentinhaber: **B.R.A.H.M.S DIAGNOSTICA GMBH**
**12099 Berlin (DE)**

(72) Erfinder: **Bergmann, Andreas, Dr.**
**D-12351 Berlin (DE)**

(74) Vertreter: **Andrae, Steffen, Dr. et al**
**Andrae Flach Haug**
**Balanstrasse 55**
**81541 München (DE)**

(56) Entgegenhaltungen:
• **CHEMICAL ABSTRACTS, Band 120, Nr. 5, 31. Jänner 1994, Columbus, Ohio, USA K. KASAGI et al. "Fundamental and clinical evaluation of an immunoradiometric assay for thyroglobulin in sera with positive autoantibodies" Seite 481, Nr. 49 426d; & Igaku to Yakugaku 1993, 30(1), 83-93**
• **CHEMICAL ABSTRACTS, Band 120, Nr. 21, 23. Mai 1994, Columbus, Ohio, USA K. SATO et al. "Measurement of thyroglobulin in blood by immunoradiometric assay" Seite 496, Nr. 264 888m; & Kaku Igaku Gijutsu 1993, 13(4), 298-303**
• **CHEMICAL ABSTRACTS, Band 102, Nr. 13, 1. April 1985, Columbus, Ohio, USA A.J. VAN HERLE et al. "An International Cooperative Study Evaluating Serum Thyro- globulin Standards" Seite 339, Nr. 109 105p; & J.Clin. Endocrinol. Metab. 1985, 60(2), 338-43**

## Beschreibung

Die vorliegende Erfindung betrifft ein Verfahren zur immunologischen Bestimmung von Proteinen oder Polypeptiden, die als Tumormarker geeignet sind, in einer Probe einer biologischen Flüssigkeit durch deren Umsetzung mit immunologischen Bindungspartnern für das zu bestimmende Protein oder Polypeptid in einer Meßlösung und Gewinnung eines Meßergebnisses, das unter Verwendung von Standardkurven, die mit Hilfe von Standardproben und eines Nullstandards erstellt werden, ausgewertet wird, wobei in der biologischen Flüssigkeit mit der Anwesenheit von weiteren Bestandteilen proteinischer oder peptidischer Natur, die zu einer methodischen Meßwertverfälschung führen, gerechnet werden muß.

Im Rahmen der klinischen Diagnostik hat der Nachweis und die quantitative Bestimmung von Proteinen und Polypeptiden, deren Auftreten in Körperflüssigkeiten von Patienten einen Hinweis auf die Existenz von Tumorgewebe verschiedenen Typs im Körper eines Patienten liefert und die daher als Tumormarker bezeichnet werden, erhebliche Bedeutung. Als derartige Tumormarker im Sinne der Verwendung dieses Begriffs in der vorliegenden Anmeldung können beispielsweise genannt werden - unter Verwendung der üblichen Abkürzungen - das prostataspezifische Antigen (PSA), das carcino-embryonale Antigen (CEA), die Tumorantigene CA 19-9, CA 1-5, CA 72-4, CA 15-3, das Alpha-Fetoprotein (AFP), PAP und andere dem Fachmann geläufige Tumorantigene proteinischer oder polypeptidischer Natur sowie insbesondere das Protein Thyreoglobulin, auf das nachfolgend noch besonders eingegangen wird und für dessen Bestimmung die vorliegende Erfindung besondere Bedeutung hat. Die vorliegende Erfindung wird daher in erster Linie anhand der Bestimmung von humanen Thyreoglobulin näher erläutert. Die Bestimmung der als Tumormarker dienenden Proteine und Polypeptide erfolgt im Rahmen der klinischen Diagnostik in erster Linie unter Verwendung von immunologischen Bestimmungsverfahren oder Immunoassays, deren wesentlichstes Kennzeichen es ist, daß man die selektiven Bindungsreaktionen der als Antigene wirkenden Tumormarker mit ihren immunologischen Bindungspartnern ausnutzt. Dem Fachmann sind heute zahlreiche verschiedene Grundverfahren für die immunologische Bestimmung von Proteinen oder Polypeptiden bzw. generell Antigenen bekannt, die teilweise, wie der klassische RIA, nach einem Konkurrenzprinzip arbeiten, indem beispielsweise die zu bestimmende Substanz sowie eine bekannte Menge der gleichen, jedoch markierten Substanz um in begrenzter Anzahl vorhandene Bindungsstellen eines immunologischen Bindungspartners, z.B. eines Antikörpers, konkurrieren, oder die sogenannte immunometrische Bestimmungen darstellen, bei denen mit markierten Bindungspartnern gearbeitet wird. Die bekannteste Variante der sogenannten immunometrischen Bestimmungen ist der klassische "Sandwich-Assay", bei dem mittels eines Überschusses eines ersten immunologischen Bindungspartners die zu bestimmende Substanz aus der Probe extrahiert und an eine Festphase gebunden wird, wonach die gesamte extrahierte Menge der zu bestimmenden Substanz durch Umsetzung mit einem weiteren markierten Bindungspartner, der an ein anderes Epitop der zu bestimmenden Substanz bindet, markiert wird. Es sind dem Fachmann zahlreiche für eine Markierung geeignete Substanzklassen und Reaktionssysteme bekannt, zu denen radioaktive Isotope, Enzyme oder Substrate einer enzymatischen Reaktion gehören oder auch Substanzen, die aufgrund ihrer Fluoreszenz oder ihres Beitrags zu einer Chemilumineszensreaktion als Marker dienen. Im Rahmen der vorliegenden Erfindung ist die Wahl eines geeigneten bekannten Markers nicht kritisch, und die vorliegende Erfindung erstreckt sich grundsätzlich auf alle bekannten oder gegebenenfalls noch gefundenen Marker.

Bei der immunologischen Bestimmung von Substanzen proteinischer oder polypeptidischer Natur durch Umsetzung mit geeigneten immunologischen Bindungspartnern ist es für die Gewinnung eines aussagekräftigen Meßwerts erforderlich, daß die dem jeweiligen Bestimmungsverfahren zugrundeliegende immunologische Bindungsreaktion zwischen den spezifischen Bindungspartnern reproduzierbar abläuft. Es gibt nunmehr jedoch Fälle, bei denen bekannt ist, daß die einem Bestimmungsverfahren zugrundeliegende immunologische Bindungsreaktion dadurch gestört werden kann, daß im Serum oder anderen Körperflüssigkeiten einzelner Patienten Bestandteile enthalten sind, die mit der immunologischen Bestimmungsreaktion interferieren. Beispiele für derartige Bestandteile sind reaktive Proteine oder Polypeptide, die ähnlich wie die zu bestimmende Substanz an irgendeinen Partner der jeweiligen immunologischen Bindungsreaktion binden und diesen damit hindern, in die immunologische Bindungsreaktion einzutreten, auf der das jeweilige Bestimmungsverfahren beruht. So können beispielsweise bei bestimmten Patienten neben den zu bestimmenden Proteinen oder Polypeptiden auch Autoantikörper gegen die gleichen Proteine oder Polypeptide vorhanden sein, die durch ihre Bindung die eigentliche Bindungsreaktion ganz oder teilweise verhindern.

Andere mögliche Bestandteile, die die Messung verfälschen können, sind Fremdproteine oder Fragmente des zu bestimmenden Proteins, die mit einem der Bindungspartner reagieren, z.B. einem für den Test vorgesehenen Antikörper, und somit ebenfalls die Bestimmung stören. Die Anwesenheit derartiger weiterer Bestandteile äußert sich in einer methodischen Meßwertverfälschung, die dazu führt, daß kein oder ein zu niedriger Meßwert erhalten wird, so daß auf der Basis des erhaltenen Meßwerts keine zuverlässige Aussage mehr gemacht werden kann.

Es ist daher wichtig, durch in der Probe vorhandene Bestandteile verursachte methodische Meßwertverfälschungen klar zu erkennen, was durch eine zusätzliche sogenannte Wiederfindungsmessung geschieht. Bei der Wiederfindungsmessung wird einer weiteren Probe der gleichen vermessenen biologischen Flüssigkeit eine geringe, bekannte Menge der zu bestimmenden Substanz zugesetzt, und die Messung wird wiederholt. Wenn das angewandte Bestimmungsverfahren für die gemessene Patientenprobe korrekte Werte liefert, muß bei der Wiederfindungsmessung die der Wiederfindungsprobe zugesetzte Menge der zu bestimmenden Substanz als eine entsprechende Erhöhung des vorher in der ersten Probe gefunden Meßwerts erscheinen. Dabei werden in der Regel Meßergebnisse innerhalb eines bestimmten normierten Bereichs um den Bereich einer 100%igen Wiederfindung (englisch: Recovery) als für eine Diagnose verwertbare positive Meßergebnisse angesehen. Da eine Fehldiagnose aufgrund einer Einzelbestimmung, die nicht durch eine Wiederfindungsmessung überprüft wurde, ernste Folgen haben kann, ist für die immunologische Bestimmung zahlreicher Substanzen, insbesondere von Tumormarkern, die Durchführung einer Wiederfindungsmessung vorgeschrieben. Das zeigt sich auch daran, daß für derartige Tests, die mit Wiederfindungs- oder Bestätigungsmessung durchgeführt werden müssen, erhöhte Gebührensätze angesetzt werden, die das Mehrfache einfacher immunologischen Untersuchungen betragen (vgl. z.B. in Deutschland die "Vertragsgebührenordnung der Kassenärztlichen Bundesvereinigung" (EPM), Pos. 4125 "humanes Thyreoglobulin mit Bestätigungstest").

Die Durchführung einer zweiten Wiederfindungs- oder Bestätigungsmessung ist mit einer Reihe von Nachteilen verbunden. Einmal verteuert der Zwang zur Durchführung der Wiederfindungs- oder Bestätigungsmessung eine diagnostische Untersuchung erheblich, zum anderen wird in der Praxis immer wieder beobachtet, daß vorschriftswidrig auf die Wiederfindungs- und Bestätigungsmessung verzichtet wird, so daß die Gefahr einer unrichtigen Interpretation des erhaltenen Meßergebnisses durch den Arzt besteht. Zum anderen wird eine Wiederfindungsmessung so durchgeführt, daß zwar einerseits ein genau gleiches Volumen einer ursprünglichen Patientenprobe wie bei der Originalmessung vermessen wird, daß die Messung jedoch aufgrund der in Form einer Lösung zugesetzten bekannten Menge der Substanz in einem etwas größeren Flüssigkeitsvolumen als die Originalmessung erfolgt. Zwei getrennte Messungen wurden bisher jedoch als unumgänglich angesehen, und die oben genannten Nachteile wurden in Kauf genommen.

Es ist Aufgabe der vorliegenden Erfindung, ein gattungsgemäßes Verfahren, d.h. ein Verfahren zur immunologischen Bestimmung von Proteinen oder Polypeptiden, die als Tumormarker geeignet sind, in einer Probe einer biologischen Flüssigkeit durch deren Umsetzung mit immunologischen Bindungspartnern für das zu bestimmende Protein oder Polypeptid in einer Meßlösung und Gewinnung eines Meßergebnisses, das unter Verwendung von Standardkurven, die mit Hilfe von Standardproben und eines Nullstandards erstellt werden, ausgewertet wird, wobei in der biologischen Flüssigkeit mit der Anwesenheit von weiteren Bestandteilen proteinischer oder peptidischer Natur, die zu einer methodischen Meßwertverfälschung führen, gerechnet werden muß, so auszugestalten, daß bei der Originalmessung direkt festgestellt werden kann, ob die durchgeführte Bestimmung mit einer methodischen Meßwertverfälschung belastet ist.

Erfindungsgemäß wird diese Aufgabe dadurch gelöst, daß man bei einem Verfahren der gattungsgemäßen Art sowohl die Bestimmung der Proteine oder Polypeptide in der Probe als auch die Erstellung der Standardkurven mit Hilfe der Standardproben und des Nullstandards in Gegenwart einer der Meßlösung von einer vornherein zugesetzten festen Menge des zu bestimmenden Proteins oder Polypeptids durchführt und daß man die Auswertung so vornimmt, daß man einen für eine Probe erhaltenen Meßwert, der oberhalb des für den Nullstandard erhaltenen Meßwerts liegt, als positiven Meßwert ansieht, der die Anwesenheit und Konzentration des zu bestimmenden Proteins oder Polypeptids anzeigt, während man einen für eine Probe erhaltenen Meßwert, der unterhalb des Meßwerts für den Nullstandard liegt, als Zeichen für eine methodische Meßwertverfälschung ansieht.

Vorzugsweise wird das erfindungsgemäße Verfahren so durchgeführt, daß man die Bestimmung in einem speziellen Probengefäß durchführt, das die zugesetzte Menge des Proteins oder Polypeptids bereits vorgelegt enthält. Das Probengefäß ist vorzugsweise ein beschichtetes Teströhrchen (coated tube, CT) oder eine beschichtete Mikrotiterplatte, die außerdem einen immobilisierten immunologischen Bindungspartner, z.B. einen Antikörper, für das zu bestimmende Protein oder Polypeptid aufweist.

Es versteht sich jedoch, daß die bekannte zuzusetzende Menge auch auf andere Weise zugesetzt werden kann, z.B. als Zusatz zu anderen Komponenten des Kits, die in festgelegten Proportionen sowohl den Standards als auch den Proben zugesetzt werden, z.B. als Zusatz zur Tracerlösung.

Vorzugsweise wählt man die bekannte zugesetzte Menge des Proteins oder Polypeptids so, daß sie einen signifikanten Basis-Meßwert (Meßwert für den Nullstandard) liefert, was erfahrungsgemäß dann gewährleistet ist, wenn die zugesetzte Menge des Proteins oder Polypeptids bei der Messung im Bereich des 5- bis 10-fachen der Menge für die untere Nachweisgrenze des Proteins oder Polypeptids im jeweiligen speziellen immunologischen Bestimmungsverfahren liegt.

Eine für die Anmelder besonders interessante Ausführungsform des erfindungsgemäßen Verfahrens betrifft die immunologische Bestimmung von humanem Thyreoglobulin. Thyreoglobulin ist ein hochmolekulares Protein, an dem die Schilddrüsenhormonsynthese stattfindet, und ist gleichzeitig der Hauptbestandteil des Schilddrüsenkolloids. Ohne daß Thyreoglobulin selbst eine direkte physiologische Wirkung aufweist, ist sein Auftreten und seine Menge im Serum ein Zeichen für die Aktivität, insbesondere Wachstumsaktivität, von Schilddrüsenzellen. Bei Gesunden liegt der Gehalt an Thyreoglobulin im Bereich von etwa 10 - 50 ng Tg/ml. Liegt ein schnell wachsendes Schilddrüsenkarzinom vor, ist der Thyreoglobulinspiegel im Serum drastisch erhöht. Von besonderer Bedeutung ist die Bestimmung von Thyreoglobulin jedoch im Zusammenhang mit der Beobachtung des Erfolgs einer operativen Entfernung eines Schilddrüsenkarzinoms, da nicht entfernte Karzinomzellen und auch als Metastasen vorhandene Zellen des Karzinomgewebes Thyreoglobulin erzeugen, das im Serum des Patienten nachweisbar ist. So sollte nach einer Totalentfernung der Schilddrüse der Thyreoglobulinwert auf 0 ng Tg/ml sinken. Ist noch Thyreoglobulin nachweisbar, bedeutet das, daß der Körper noch Schilddrüsengewebe, evtl. in Form von Metastasen des Karzinoms, enthält.

Bei der Bestimmung von humanem Thyreoglobulin ist eine Wiederfindungsmessung deshalb bisher unbedingt erforderlich, weil bei einem signifikanten Prozentsatz von Patienten gleichzeitig Bestandteile auftreten, die zu einem methodischen Meßfehler der oben beschriebenen Art im Sinne einer Erniedrigung des Meßwerts für die in der Probe tatsächlich vorhandene Konzentration der zu bestimmenden Substanz führen. Derartige Bestandteile sind insbesondere anti-Tg-Autoantikörper, es ist jedoch davon auszugehen, daß die Proben auch noch andere Bestandteile enthalten können, die die Bestimmung stören, da nicht immer eine Parallelität zwischen den Ergebnissen von Wiederfindungsmessung und einer direkten Bestimmung der Menge an anti-Tg-Autoantikörpern festgestellt wird. Als derartige andere Bestandteile werden z.B. proteinische oder polypeptidische Bruchstücke des Thyreoglobulins diskutiert.

Wenn im Rahmen der vorliegenden Erfindungsbeschreibung Thyreoglobulin vorwiegend als Tumormarker bezeichnet wird, ist damit keinerlei Einschränkung des erfindungsgemäßen Verfahrens auf irgendeinen bestimmten Meßzweck beabsichtigt, sondern jede Bestimmung von Thyreoglobulin nach einem Verfahren, das die erfindungsgemäßen Merkmale aufweist, wird von der vorliegenden Erfindung umfaßt. Dasselbe gilt für die Messung anderer Tumormarker, wenn diese auch noch zu anderen Zwecken als zum Nachweis eines Tumors bestimmt werden.

Gemäß der vorliegenden Erfindung wurde überraschend festgestellt, daß ohne signifikante Einbußen an Meßgenauigkeit auf die Wiederfindungsmessung bei der Bestimmung von humanem Thyreoglobulin (hTg) verzichtet werden kann, wenn man alle Bestimmungen sowie auch die Erstellung der Standardkurven in Gegenwart einer von vornherein vorgelegten festen Zusatzmenge an humanem Thyreoglobulin durchführt, d.h. unter Bedingungen, unter denen der Meßwert für das Basissignal für hTg künstlich erhöht ist. Es zeigte sich, daß eine vorgelegte Menge an Thyreoglobulin keinen Einfluß auf die Genauigkeit der Bestimmung der Thyreoglobulin-Konzentration in der untersuchten Probe hat, wenn die Bestimmung durch keine methodische Meßwertverfälschung belastet ist, so daß das Bestimmungsverfahren in einem solchen Falle genauso exakt ist, wie das bisher für die Originalbestimmung verwendet Verfahren.

Sind in der biologischen Probe jedoch Substanzen vorhanden, die das immunologische Bestimmungsverfahren stören, indem sie beispielsweise bei einem immunometrischen Bestimmungsverfahren nach der Sandwich-Methode die korrekte Ausbildung des Sandwiches verhindern, äußert sich das bei dem erfindungsgemäßen Verfahren dann, wenn die Probe kein dem Patienten entstammendes Thyreoglobulin enthält, auf jeden Fall als Ermittlung eines zu geringen Meßwerts für die zugesetzte Thyreoglobulinmenge, d.h. als Meßwert, der unter dem für den Nullstandard liegt.

Wenn die Patientenprobe gleichzeitig sowohl Thyreoglobulin als auch Bestandteile enthält, die zu einer methodischen Meßwertverfälschung führen, überlagern sich beide Effekte. Der aus der Bestimmung erhaltene Meßwert ist jedoch trotzdem in der Regel von hoher klinischer Wertigkeit: Wird eine Abweichung von dem für die Zusatzmenge zu erwartenden Meßwert nach unten beobachtet, ist das ein sicherer Hinweis auf eine methodische Meßwertverfälschung, und der entsprechende Wert ist zu verwerfen bzw. nach einem anderen Verfahren zu überprüfen. Wird eine Thyreoglobulinmenge ermittelt, die über der Erwartungsmenge (bei Gesunden 10 - 50 ng Tg/ml, nach Totaloperation 0 ng Tg/ml) liegt, ist das auf jeden Fall ein Hinweis auf einen erhöhten Thyreoglobulinspiegel im untersuchten Serum, so daß auf jeden Fall eine wenigstens qualitative Aussage gemacht werden kann und ggf. weitere Untersuchungen veranlaßt werden können.

Das erfindungsgemäße Verfahrens bietet stets dann einen Vorteil, wenn auf eine Wiederfindungsmessung verzichtet wurde, da die Gefahr, einen unrichtigen Meßwert als korrekten anzusehen, durch die deutliche Erkennbarkeit methodischer Meßfehler in der Originalmessung stark verringert ist. Das erfindungsgemäße Verfahren läßt sich ferner in Zweifelsfällen auf einfache Weise als Bestätigungsmessung durchführen. In diesem Falle wiederholt man den Test einfach mit einer anderen Verdünnung der Patientenprobe, wobei man im gleichen Testvolumen arbeitet. Da sich durch die Verdünnung der Patientenprobe die Relation von fester Zusatzmenge zu den Bestandteilen der Patientenprobe verändert, wird ein gegebenenfalls vorher maskierter Meßwert bei der anderen Verdünnung als Abweichung von dem Erwartungswert für die Zusatzmenge erkennbar. Das Verfahren hat auch in diesem Fall den zusätzlichen Vorteil, daß mit gleichen Probenvolumina gearbeitet werden kann, d.h. eine denkbare Beeinflussung des Meßwerts durch die bei den bisherigen Wiederfindungsmessungen erhöhten Volumina der Meßlösungen vermieden werden kann.

Das erfindungsgemäße Verfahren bietet ferner den erheblichen praktischen Vorteil, daß es auch vollautomatisiert auf üblichen Vollautomaten zu Durchführungen immunologischer Bestimmungen durchgeführt werden kann, für die die bisher erforderlichen Wiederfindungsmessungen ein unlösbares Problem darstellten. In einem solchen Falle muß nicht unbedingt mit Teströhrchen gearbeitet werden, die die Zusatzmenge in lyophilisierter Form als Wandbeschichtung enthalten, sondern es ist dann auch möglich, die feste Zusatzmenge in Form einer Lösung durch den Vollautomaten in alle Meßröhrchen geben zu lassen.

Wie das nachfolgende Anwendungsbeispiel zeigt, erweist sich in der Praxis das erfindungsgemäße Verfahren in Einzelfällen sogar als genauer als das bisherige, mit Wiederfindungsmessung durchzuführende Bestimmungsverfahren, indem es eine sicherere Unterscheidung zwischen korrekten und unkorrekten Meßwerten in der Grauzone, in der Zweifel an der Korrektheit eines erhaltenen Meßergebnisses bestehen, ermöglicht.

Die Durchführbarkeit und Zuverlässigkeit des erfindungsgemäßen Verfahrens wurde im Vergleich mit einem kommerziellen Assay der Anmelderin, der als DYNOtest® Tg vertrieben wird, untersucht, wobei das erfindungsgemäße Verfahren als Ausgestaltung des genannten Assays direkt auf diesem aufbaut.

Zur Erläuterung wird Bezug genommen auf zwei Tabellen und drei Figuren mit graphischen Darstellungen, die zeigen:

Fig. 1 zeigt typische Standardkurven für die Bestimmung von hTg nach dem bekannten Test (b) und nach dem erfindungsgemäßen Verfahren (a);

Fig. 2 zeigt die Korrelation des bekannten Tests mit dem erfindungsgemäßen Test für Patientenproben mit korrekter Wiederfindung, und

Fig. 3 zeigt eine entsprechende Korrelation für Proben, bei denen aufgrund der Wiederfindungsversuche meßwertverfälschende Bestandteile in der Patientenprobe angenommen werden mußten.

Der bekannte DYNOtest® Tg ist ein immunoradiometrischer Assay zur Bestimmung von Thyreoglobulin (Tg) im Humanserum. Dabei werden zwei antigenspezifische monoklonale Antikörper, die das hTg (als Antigen) an jeweils verschiedenen Determinanten erkennen, im Überschuß eingesetzt. Einer der beiden Antikörper ist radioaktiv markiert (Tracer), der andere ist auf der Innenseite der Teströhrchen fixiert (coated-tube-Technik).

Im Verlaufe der Inkubation der Probe mit den Assay-Reagenzien reagieren beide Antikörper nacheinander mit den hTg-Molekülen der Probe, wobei ein "Sandwich-Komplex" entsteht, der auf der Röhrchenoberfläche haftet. Nach Reaktionsende wird der verbliebene Tracerüberschuß in der Flüssigphase durch Absaugen oder Dekantieren entfernt und verworfen.

Nach zweimaligem Waschen wird dann die Radioaktivität der Röhrchen gemessen. Sie ist der hTg-Konzentration der jeweiligen Probe bei Abwesenheit störender Bestandteile direkt proportional. Anhand der dem Assay beigegebenen Standards und eines Nullstandards wird eine Standardkurve erstellt, woraus über die für die einzelnen Proben gemessenen Radioaktivitäten der Patientenseren die Konzentration des darin enthaltenen hTg ermittelt wird.

Ein Assay der genannten Art wird als Kit (Reagenziensatz) vertrieben, der folgende Komponenten, in Mengen ausreichend für 100 (2 x 50) Bestimmungen, enthält:

1. 125 I-anti-hTg-Antikörper (monoklonal; Maus) als radioaktiven Tracer in zwei Fläschchen ä 10,5 ml, gebrauchsfertig, Aktivität jeweils ca. 225 kBq (bei 70% Zählausbeute) entsprechend ca. 180.000 IpM/200µl

2. Coated tubes (Teströhrchen) beschichtet mit anti-hTg-Antikörper (monoklonal; Maus); 2 x 50 Stück; gebrauchsfertig

3. 1 Fläschchen ä 3 ml Humanserum als hTg-Nullstandard, gebrauchsfertig; definiert als 0,2 ng Tg/ml. Der Nullstandard ist auch als Verdünnungsmittel für Patientenseren vorgesehen, wenn relativ hohe hTg-Konzentrationen zu erwarten sind.

4. hTg-Standards (Humanserum), 6 Fläschchen ä 0,4 ml; gebrauchsfertig; Konzentrationen, 1,6; 3,1; 12,5; 50; 200; 500 ng Tg/ml

5. Tg-Wiederfindungsprobe, 1 Fläschchen ä 0,7 ml; gebrauchsfertig; Konzentration: 500 ng Tg/ml. Pro Wiederfindungsmessung werden davon 10 µl in die Meßlösung pipettiert.

6. Waschlösung in 2 Fläschchen ä 10 ml als Konzentrat, das vor Gebrauch mit destilliertem Wasser auf jeweils 500 ml aufzufüllen ist.

7. Drei Kontrollseren I, II und III (Humanserum) in 3 Fläschchen ä 0,4 ml; gebrauchsfertig.

Testdurchführung:

Bei einem Test werden in die gekennzeichneten Teströhrchen jeweils 50 µl Tg-Standard pipettiert, und in die Röhrchen für die Proben werden je 50 µl Patientenserum pipettiert.

Für den Nullstandard und die Patientenproben ist ein paralleler Wiederfindungsversuch erforderlich, wozu ein weiterer Satz Teströhrchen mit 50 µl Patientenserum mit jeweils 10 µl Wiederfindungsprobe versetzt werden. Auch ein Röhrchen mit 50 µl Nullstandard wird mit 10 µl Wiederfindungsprobe versetzt.

Anschließend wird in jedes Teströhrchen 200 µl Tracer pipettiert, und nach einer Inkubation über Nacht bei Raumtemperatur werden die Teströhrchen mit Waschlösung versetzt, dekantiert oder abgesaugt, wobei das Waschen zweimal wiederholt wird.

Anschließend wird die Radioaktivität eines jeden Röhrchens in einem gamma-Counter gemessen.

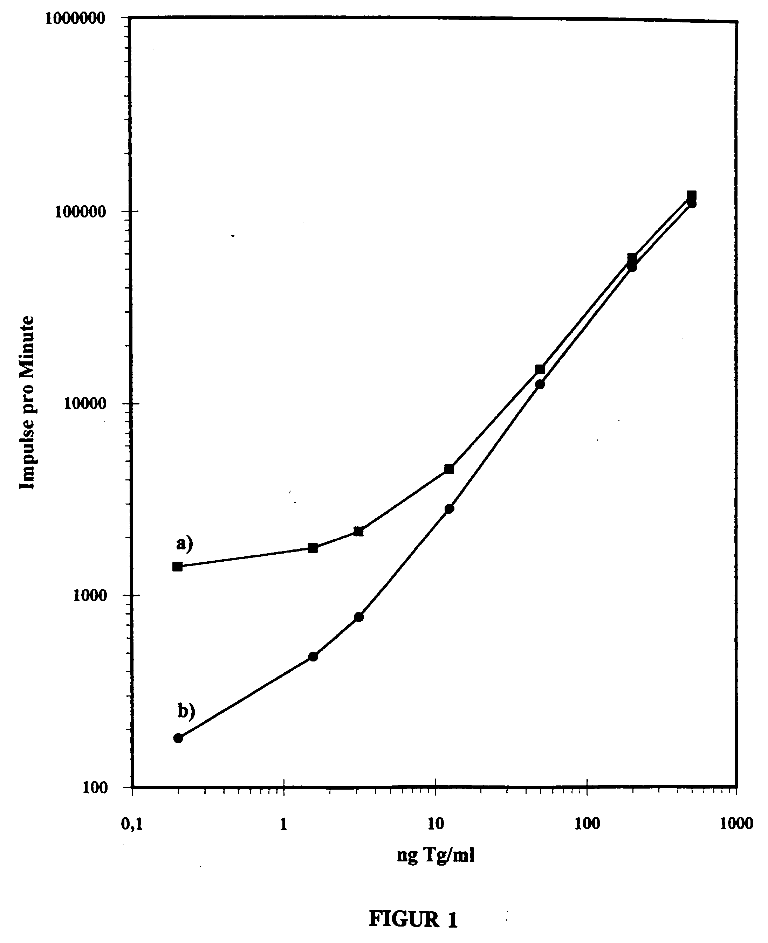

Aus den Messungen der sechs Standardproben wird eine Standardkurve erhalten, und der Meßwert für die Patientenseren wird unter Bezugnahme auf die genannte Standardkurve ausgewertet. Eine typische Standardkurve für den bekannten DYNOtest® Tg ist als Kurve b) in Figur 1 gezeigt.

Der vorgeschriebene Wiederfindungsversuch dient dazu, methodische Verfälschungen des Meßwerts zu entdecken. Bei ungestörter Wiederfindung, d.h. wenn sich keine die hTg-Bestimmung verfälschenden Faktoren im Patientenserum finden, muß der hTg-Wert im Wiederfindungsversuch um etwa 100 ng/ml (entsprechend der zugesetzten Wiederfindungsprobe) höher liegen als der hTg-Wert der parallel bestimmten Originalserumprobe.

Die Wiederfindung errechnet sich nach der folgenden Formel

$$\frac{\text{ng Tg/ml (W) - ng Tg/ml (Probe)}}{100 \text{ ng Tg/ml}} \times 100 = \% \text{ Wiederfindung}$$

In der obigen Formelgleichen steht (W) für das Meßergebnis einer Patientenprobe unter Zusatz der Wiederfindungsprobe und (Probe) steht für den Meßwert einer Originalprobe.

Bei dem Verfahren wird weiterhin empfohlen, die methodische Verläßlichkeit der Durchführung der Wiederfindung zu überprüfen. Dazu wird ein zusätzlicher Wiederfindungsversuch für den Nullstandard durchgeführt, der ca. 100 ng Tg/ml ergeben muß, entsprechend 100 % Wiederfindung.

Der Toleranzbereich für die korrekte Wiederfindung liegt zwischen 70 bis 130 % oder 80 bis 120 %.

Bei dem DYNOtest® Tg-Assay wird eine untere Nachweisgrenze von < 1 ng Tg/ml als Assayempfindlichkeit erhalten.

Erfindungsgemäßes Verfahren

Zur Durchführung und Überprüfung des erfindungsgemäßen Verfahrens wurde der Kit (Reagenziensatz) bzw. das Meßprotokoll des obigen DYNOtest® Tg dadurch abgewandelt, daß in alle Teströhrchen, die bereits mit dem monoklonalen anti-hTg-Antikörper beschichtet waren, eine Lösung von 0,3 ng hTg in 100 µl phosphatgepufferter Salzlösung (PBS) mit einem Gehalt an 1 % bovinem Serumalbumin (BSA) pipettiert wurde. Durch anschließende Lyophilisierung wurde die in die Teströhrchen gegebene Menge an hTg in die Beschichtung der Teströhrchenwände überführt.

Anstelle einer Verwendung der mit hTg beschichteten Röhrchen liegt es jedoch im Bereich der vorliegenden Erfindung, die feste Zusatzmenge einer anderen, bei jeder einzelnen Bestimmung in im wesentlichen identischer Form zu verwendenden Testkomponente zuzugeben, insbesondere der Tracerlösung.

Ein Reagenziensatz für die Durchführung des erfindungsgemäßen Verfahrens umfaßt in keinem der genannten Fälle eine gesonderte Tg-Wiederfindungsprobe.

Es wurde in einem Parallelversuch nach dem erfindungsgemäßen Verfahren auf übliche Weise, jedoch in Anwesenheit der zugesetzten Menge an hTg, eine Standardkurve (Kurve a) in Figur 1) erstellt, die der für den kommerziellen DYNotest® Tg entsprach, und beide Standardkurven wurden zur Auswertung von 179 Patientenseren herangezogen, die jeweils in beiden Assaysystemen vermessen wurden.

Die nachfolgende Tabelle 1 gibt die Daten für die Erstellung der beiden Standardkurven a) und b) in Figur 1 wieder, und zwar in IpM (Impulse pro Minute).

Tabelle 1

|         | Konz. in ng Tg/ml | IpM a) | IpM b) |
|---------|-------------------|--------|--------|
| Std. 0  | 0                 | 1410   | 181    |
| Std. 1  | 1,563             | 1754   | 477    |
| Std. 2  | 3,125             | 2147   | 770    |
| Std. 3  | 12,5              | 4543   | 2819   |
| Std. 4  | 50                | 15049  | 12621  |

Tabelle 1 (fortgesetzt)

|  | Konz. in ng Tg/ml | IpM a) | IpM b) |
|---|---|---|---|
| Std. 5 | 200 | 57698 | 51645 |
| Std. 6 | 500 | 123615 | 112313 |

Tabelle 2 zeigt die erhaltenen Ergebnisse, und zwar in der ersten Spalte die willkürliche Patientenkennziffer, in der zweiten Spalte den nach dem erfindungsgemäßen Verfahren erhaltenen Meßwert, in der dritten Spalte den nach dem bekannten Verfahren erhaltenen Meßwert sowie in der vierten Spalte die errechnete Wiederfindung für das bekannte Verfahren.

Die durch Auswertung der ursprünglichen Meßsignale erhaltenen Meßergebnisse sind angegeben als ng/ml mit Ausnahme derjenigen Fälle, bei denen in dem erfindungsgemäßen Test anhand einer Erniedrigung des Meßwerts für die zugesetzte Tg-Menge das Auftreten einer systematischen Meßwertverfälschung festgestellt wurde. In diesen Fällen ist das Meßergebnis als Prozentsatz des für die Zusatzmenge erwarteten Basis-Meßwerts angegeben.

In Tabelle 2 sind zwei Klassen von Patientenseren zu erkennen, nämlich solche, bei denen im bekannten Test im Wiederfindungsversuch die Korrektheit der Probenmessung bestätigt wurde, sowie solche, bei denen im Wiederfindungsversuch das Auftreten einer systematischen Meßwertverfälschung festgestellt wurde.

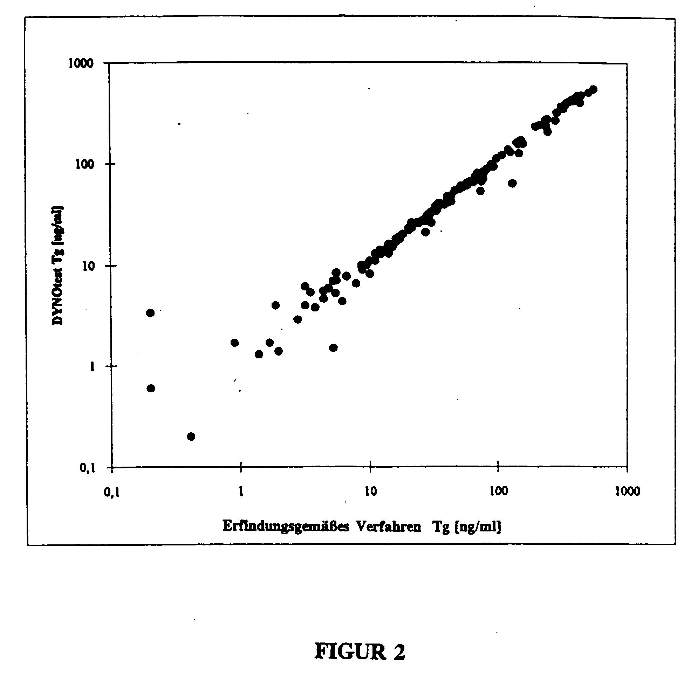

Fig. 2 zeigt, daß eine hervorragende Korrelation zwischen den Ergebnissen des bekannten DYNOtest® sowie des erfindungsgemäßen Tests bestand, wobei der Korrelationskoeffizient 0,99 betrug.

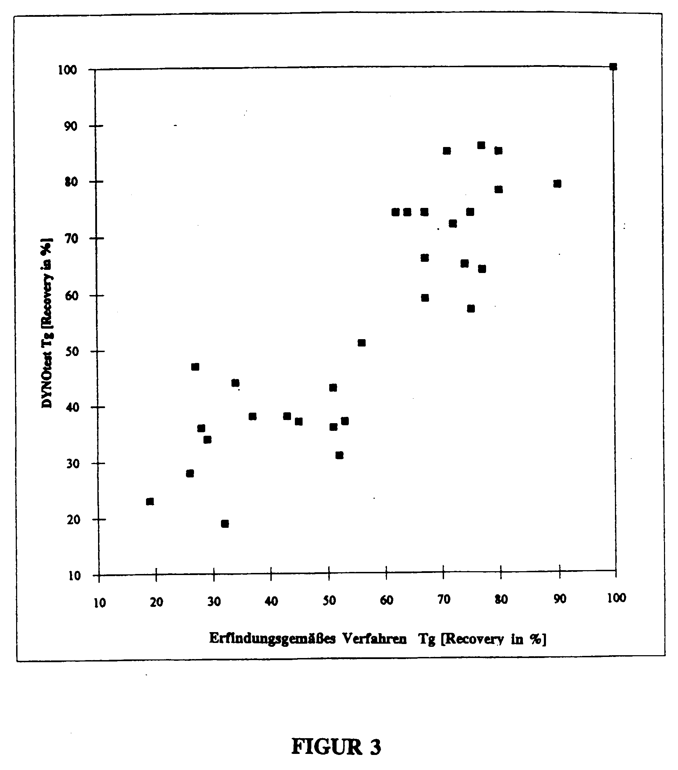

Fig. 3 zeigt ferner, daß auch bei den Proben mit nichtkorrekter Wiederfindung im wesentlichen eine gute Korrelation zwischen den beiden Tests bestand (Korrelationskoeffizient 0,79), daß nach dem erfindungsgemäßen Verfahren jedoch eine ganze Reihe von Proben, die nach dem bekannten Test aufgrund einer Wiederfindung von mehr als 70 % als noch akzeptable Meßergebnisse zu gelten hatten, bei dem erfindungsgemäßen Verfahren aufgrund einer stärkeren Erniedrigung des Meßwerts für das Basissignal deutlich als mit einem Meßfehler behaftet erschienen.

Während bei dem bekannten Verfahren die gezeigten Werte mit zwei Messungen pro Probe erhalten wurden, d.h. nämlich für die Originalprobe und Probe + Wiederfindung, wurden nach dem erfindungsgemäßen Verfahren entsprechende Meßergebnisse von gleicher Präzision und Aussagekraft mit einer einzigen Bestimmung erhalten.

Damit erweist sich das erfindungsgemäße Verfahren als ein dem bekannten Verfahren im Hinblick auf die Meßgenauigkeit äquivalentes Verfahren, hat darüber hinaus jedoch den Vorteil, daß bei Meßwerten, die nach dem bekannten Verfahren nur die Aussage "kein oder so gut wie kein Tg" (Meßwerte im Bereich von 0 ng Tg/ml in der Tabelle) liefern, bei dem erfindungsgemäßen Verfahren gleichzeitig festgestellt wird, ob der erhaltene Meßwert korrekt sein kann oder ob er aufgrund einer festgestellten Erniedrigung des Basis-Meßwerts für die Zusatzmenge in einer mit einem methodischen Fehler behafteten Messung ermittelt wurde.

Tabelle 2

| Patient | Erfindungsgemäßes Verfahren | DYNOtest Tg | Recovery |
|---|---|---|---|
|  | [ng Tg/ml] | [ng Tg/ml] | [%] |
|  |  |  |  |
| 1 | 546 | 555 | 96 |
| 2 | 439 | 482 | 95 |
| 3 | 431 | 475 | 97 |
| 4 | 409 | 476 | 82 |
| 5 | 379 | 420 | 96 |
| 6 | 375 | 437 | 94 |
| 7 | 354 | 415 | 87 |
| 8 | 336 | 402 | 97 |
| 9 | 327 | 377 | 99 |
| 10 | 319 | 354 | 99 |
| 11 | 308 | 371 | 104 |

Tabelle 2   (fortgesetzt)

| Patient | Erfindungsgemäßes Verfahren | DYNOtest Tg | Recovery |
|---|---|---|---|
| | [ng Tg/ml] | [ng Tg/ml] | [%] |
| 12 | 285 | 324 | 104 |
| 13 | 278 | 269 | 109 |
| 14 | 243 | 209 | 95 |
| 15 | 241 | 278 | 108 |
| 16 | 236 | 238 | 104 |
| 17 | 235 | 276 | 100 |
| 18 | 228 | 244 | 108 |
| 19 | 212 | 244 | 100 |
| 20 | 196 | 235 | 103 |
| 21 | 157 | 138 | 98 |
| 22 | 152 | 172 | 102 |
| 23 | 147 | 127 | 91 |
| 24 | 146 | 165 | 101 |
| 25 | 145 | 156 | 98 |
| 26 | 141 | 161 | 101 |
| 27 | 131 | 63 | 94 |
| 28 | 126 | 130 | 95 |
| 29 | 108 | 121 | 91 |
| 30 | 98 | 112 | 93 |
| 31 | 93 | 93 | 96 |
| 32 | 90 | 95 | 93 |
| 33 | 89 | 98 | 103 |
| 34 | 88 | 95 | 100 |
| 35 | 82 | 88 | 88 |
| 36 | 78 | 81 | 91 |
| 37 | 77 | 71 | 95 |
| 38 | 77 | 83 | 97 |
| 39 | 74 | 66 | 97 |
| 40 | 73 | 53 | 96 |
| 41 | 69 | 80 | 93 |
| 42 | 67 | 75 | 96 |
| 43 | 64 | 65 | 100 |
| 44 | 62 | 66 | 91 |
| 45 | 61 | 67 | 93 |
| 46 | 60 | 66 | 95 |
| 47 | 60 | 66 | 95 |

Tabelle 2   (fortgesetzt)

| Patient | Erfindungsgemäßes Verfahren | DYNOtest Tg | Recovery |
| --- | --- | --- | --- |
| | [ng Tg/ml] | [ng Tg/ml] | [%] |
| 48 | 58 | 65 | 96 |
| 49 | 58 | 62 | 94 |
| 50 | 57 | 64 | 94 |
| 51 | 57 | 63 | 96 |
| 52 | 57 | 61 | 96 |
| 53 | 53 | 58 | 99 |
| 54 | 51 | 60 | 96 |
| 55 | 51 | 60 | 86 |
| 56 | 51 | 57 | 93 |
| 57 | 50 | 56 | 89 |
| 58 | 43 | 42 | 87 |
| 59 | 43 | 48 | 97 |
| 60 | 40 | 47 | 92 |
| 61 | 40 | 44 | 88 |
| 62 | 40 | 42 | 91 |
| 63 | 39 | 40 | 95 |
| 64 | 38 | 19 | 96 |
| 65 | 35 | 40 | 91 |
| 66 | 34 | 37 | 96 |
| 67 | 34 | 40 | 39 |
| 68 | 33 | 36 | 95 |
| 69 | 33 | 36 | 97 |
| 70 | 33 | 34 | 91 |
| 71 | 32 | 37 | 87 |
| 72 | 32 | 35 | 93 |
| 73 | 30 | 33 | 93 |
| 74 | 30 | 26 | 72 |
| 75 | 30 | 33 | 94 |
| 76 | 29 | 32 | 95 |
| 77 | 29 | 31 | 96 |
| 78 | 28 | 31 | 93 |
| 79 | 28 | 31 | 93 |
| 80 | 27 | 29 | 92 |
| 81 | 27 | 28 | 92 |
| 82 | 27 | 27 | 96 |
| 83 | 27 | 21 | 98 |

Tabelle 2 (fortgesetzt)

| Patient | Erfindungsgemäßes Verfahren | DYNOtest Tg | Recovery |
|---|---|---|---|
| | [ng Tg/ml] | [ng Tg/ml] | [%] |
| 84 | 25 | 27 | 87 |
| 85 | 24 | 26 | 91 |
| 86 | 23 | 26 | 94 |
| 87 | 21 | 23,3 | 91 |
| 88 | 21 | 26 | 96 |
| 89 | 20 | 22 | 97 |
| 90 | 20 | 22 | 89 |
| 91 | 18 | 20 | 92 |
| 92 | 17 | 19 | 89 |
| 93 | 17 | 19 | 95 |
| 94 | 17 | 18 | 97 |
| 95 | 17 | 19 | 97 |
| 96 | 16 | 18 | 89 |
| 97 | 16 | 17 | 94 |
| 98 | 15 | 15 | 97 |
| 99 | 14 | 16 | 90 |
| 100 | 14 | 13 | 94 |
| 101 | 12.3 | 13 | 94 |
| 102 | 11 | 11 | 94 |
| 103 | 10 | 11 | 95 |
| 104 | 10 | 8,2 | 93 |
| 105 | 9,4 | 10 | 99 |
| 106 | 8,7 | 9 | 95 |
| 107 | 8,6 | 9,5 | 95 |
| 108 | 7,8 | 6,6 | 87 |
| 109 | 6,1 | 4,4 | 86 |
| 110 | 5,5 | 7,1 | 97 |
| 111 | 5,4 | 5,3 | 73 |
| 112 | 5,2 | 7 | 93 |
| 113 | 5,2 | 1,5 | 65 |
| 114 | 4,8 | 5,9 | 89 |
| 115 | 4,4 | 5,6 | 91 |
| 116 | 4,4 | 4,7 | 61 |
| 117 | 4,4 | 4,7 | 93 |
| 118 | 3,8 | 3,8 | 91 |
| 119 | 3,2 | 6,2 | 33 |

Tabelle 2   (fortgesetzt)

| Patient | Erfindungsgemäßes Verfahren | DYNOtest Tg | Recovery |
|---|---|---|---|
| | [ng Tg/ml] | [ng Tg/ml] | [%] |
| 120 | 2,8 | 2,9 | 96 |
| 121 | 2 | 1,4 | 101 |
| 122 | 1,9 | 4 | 89 |
| 123 | 1,4 | 1,3 | 55 |
| 124 | 0,9 | 1,7 | 83 |
| 125 | 0,4 | 0,2 | 85 |
| 126 | 0,2 | 3,4 | 41 |
| 127 | 0,2 | 0,6 | 50 |
| 128 | 0 | 0 | 92 |
| 129 | 1,7 | 1,7 | 88 |
| 130 | 11 | 13 | 91 |
| 131 | 13 | 14 | 95 |
| 132 | 12 | 14 | 92 |
| 133 | 46 | 54 | 98 |
| 134 | 20 | 23 | 86 |
| 135 | 8,6 | 10 | 90 |
| 136 | 12 | 13 | 89 |
| 137 | 14 | 15 | 91 |
| 138 | 6,6 | 7,8 | 88 |
| 139 | 23 | 26 | 86 |
| 140 | 500 | 513 | 95 |
| 141 | 121 | 138 | 96 |
| 142 | 367 | 423 | 89 |
| 143 | 429 | 407 | 91 |
| 144 | 0 | 0,9 | 59 |
| 145 | 3,2 | 4 | 61 |
| 146 | 3,5 | 5,4 | 76 |
| 147 | 3,5 | 8,4 | 73 |
| Patient | Erfindungsgemäßes Verfahren | DYNOtest Tg [ng Tg/ml] | Recovery [%] |
| | Meßwerte < 0 Std. in % | | |
| 148 | 28% | 0 | 36 |
| 149 | 77% | 0 | 86 |
| 150 | 37% | 0 | 38 |
| 151 | 64% | 0 | 74 |
| 152 | 56% | 0 | 51 |

Tabelle 2   (fortgesetzt)

| Patient | Erfindungsgemäßes Verfahren | DYNOtest Tg | Recovery |
| | | [ng Tg/ml] | [%] |
| | Meßwerte < 0 Std. in % | | |
|---|---|---|---|
| 153 | 52% | 0,5 | 31 |
| 154 | 27% | 0 | 47 |
| 155 | 32% | 2 | 19 |
| 156 | 19% | 2,8 | 23 |
| 137 | 51% | 3,7 | 43 |
| 158 | 77% | 0 | 64 |
| 159 | 29% | 0 | 34 |
| 160 | 26% | 0 | 28 |
| 161 | 34% | 0 | 44 |
| 162 | 75% | 0,7 | 57 |
| 163 | 67% | 0,4 | 59 |
| 164 | 67% | 0 | 66 |
| 165 | 71% | 0 | 85 |
| 166 | 62% | 0 | 74 |
| 167 | 53% | 0 | 37 |
| 168 | 45% | 0 | 37 |
| 169 | 80% | 0 | 85 |
| 170 | 80% | 0 | 78 |
| 171 | 72% | 0 | 72 |
| 172 | 67% | 0 | 74 |
| 173 | 43% | 0 | 38 |
| 174 | 75% | 0 | 74 |
| 175 | 74% | 0 | 65 |
| 176 | 98% | 0 | 79 |
| 177 | 67% | 0,2 | 66 |
| 178 | 51% | 0 | 36 |
| 179 | 100% | 0 | 100 |
| Std. steht für den Meßwert für den Nullstandard | | | |

Bei der Bestimmung von Tg nach dem erfindungsgemäßen Verfahren unter Verwendung von Teströhrchen, die von vornherein ein feste vorgegebene Menge an Tg enthalten, wurde deutlich, daß sich die Vorteile derartiger vorbeschichteter Teströhrchen auch in dem herkömmlichen Test mit gesonderter Wiederfindung nutzen lassen, indem man einem entsprechenden Kit für die Wiederfindung anstelle einer Tg-Lösung, die den Wiederfindungsproben zuzusetzen ist, einen besonderen Satz von Teströhrchen beigibt, der die Zusatzmenge für die gesonderte Wiederfindungsmessung als Beschichtung enthält. Auch in diesem Falle ergeben sich gegenüber dem herkömmlichen Verfahren einige erhebliche praktische Vorteile, nämlich das Arbeiten in gleichen Flüssigkeitsvolumina bei Bestimmung und Wiederfindungsmessung, die Vermeidung zusätzlicher Fehlerquellen beim Pipettieren der zusätzlichen Wiederfindungsprobe sowie die Möglichkeit einer automatisierten Bestimmung. Durch eine geeignete Kennzeichnung der Röhrchen für die Wiederfindungsmessung, z.B. durch eine andere Einfärbung oder eine deutliche Markierung solcher Röhrchen, können mögliche Fehlerquellen aufgrund von Verwechslungen der Teströhrchen sicher ausgeschlossen werden.

Für die Durchführung von gesonderten Wiederfindungsmessungen bestimmte Teströhrchen unterscheiden sich von den Teströhrchen, die für die Durchführung des eingangs geschilderten Bestimmungsverfahrens ohne gesonderte Wiederfindungsmessung bestimmt sind, im wesentlichen nur durch eine in der Regel höhere Menge an Thyreoglobulin pro Teströhrchen (CT). Während für das eingangs geschilderte erfindungsgemäße Verfahren die pro Teströhrchen zugesetzte Tg-Menge vorzugsweise im Bereich von 0,1 bis 0,5 ng/CT liegt, liegt sie bei Röhrchen für die gesonderte Durchführung einer Wiederfindungsmessung bei etwa der 10fachen Menge, wobei eine Menge von 5 ng/CT besonders bevorzugt ist. Die Herstellung beider Sorten von Röhrchen erfolgt, abgesehen vom Einsatz unterschiedlicher Tg-Mengen, auf im wesentlichen identische Weise.

Bei der Messung von 100 Patientenseren in teilweise unterschiedlicher Verdünnung unter Durchführung einer zusätzlichen gesonderten Wiederfindungsmessung einmal nach dem herkömmlichen Verfahren und einmal unter Verwendung von mit Tg vorbeschichteten Teströhrchen wurde eine hervorragende Übereinstimmung der Meßergebnisse erhalten. Bei der Messung von Seren ohne gestörte Wiederfindung betrug der Mittelwert für die Wiederfindung nach dem herkömmlichen Verfahren 94,5 %, nach der neuen Verfahrensvariante unter Verwendung von mit Tg vorbeschichteten Teströhrchen 96,2 %. Bei der Messung von Seren mit gestörter Wiederfindung (49 Seren) betrug der Korrelationskoeffizient beider Verfahrensvarianten 0,88.

## Patentansprüche

1.  Verfahren zur immunologischen Bestimmung von Proteinen oder Polypeptiden, die als Tumormarker geeignet sind, in einer Probe einer biologischen Flüssigkeit durch deren Umsetzung mit immunologischen Bindungspartnern für das zu bestimmende Protein oder Polypeptid in einer Meßlösung und Gewinnung eines Meßergebnisses, das unter Verwendung von Standardkurven, die mit Hilfe von Standardproben und eines Nullstandards erstellt werden, ausgewertet wird, wobei in der biologischen Flüssigkeit mit der Anwesenheit von weiteren Bestandteilen proteinischer oder peptidischer Natur, die zu einer methodischen Meßwertverfälschung führen, gerechnet werden muß, **dadurch gekennzeichnet, daß** man sowohl die Bestimmung der Proteine oder Polypeptide in der Probe als auch die Erstellung der Standardkurven mit Hilfe der Standardproben und des Nullstandards in Gegenwart einer der Meßlösung von einer vornherein zugesetzten festen Menge des zu bestimmenden Proteins oder Polypeptids durchführt und daß man die Auswertung so vornimmt, daß man einen für eine Probe erhaltenen Meßwert, der oberhalb des für den Nullstandard erhaltenen Meßwerts liegt, als positiven Meßwert ansieht, der die Anwesenheit und Konzentration des zu bestimmenden Proteins oder Polypeptids anzeigt, während man einen für eine Probe erhaltenen Meßwert, der unterhalb des Meßwerts für den Nullstandard liegt, als Zeichen für eine methodische Meßwertverfälschung ansieht.

2.  Verfahren nach Anspruch 1, dadurch gekennzeichnet, daß man die Bestimmung der Proben und die Umsetzungen für die Erstellung der Standardkurven in einem Probengefäß durchführt, das die zugesetzte Menge des Proteins oder Polypeptids als Wandbeschichtung vorgelegt enthält.

3.  Verfahren nach Anspruch 2, dadurch gekennzeichnet, daß das Testgefäß ein beschichtetes Teströhrchen (CT) oder eine beschichtete Mikrotiterplatte ist, die außerdem einen immobilisierten immunologischen Bindungspartner für das zu bestimmende Protein oder Polypeptid aufweisen.

4.  Verfahren nach Anspruch 1, 2 oder 3, dadurch gekennzeichnet, daß das zu bestimmende Protein Thyreoglobulin (Tg) ist.

5.  Verfahren nach einem der Ansprüche 1 bis 4, dadurch gekennzeichnet, daß man die zugesetzte Menge des Proteins oder Polypeptids so wählt, daß sie bei der Messung im Bereich der 5- bis 10-fachen Menge für die untere Nachweisgrenze für das zu bestimmende Protein oder Polypeptid im jeweiligen speziellen immunologischen Bestimmungsverfahren liegt.

6.  Verfahren nach den Ansprüchen 4 oder 5, dadurch gekennzeichnet, daß das Bestimmungsverfahren ein an sich bekanntes immunometrisches Bestimmungsverfahrens unter Verwendung von zwei monoklonalen, im Überschuß eingesetzten Antikörpern ist, die an verschiedene Epitope des zu bestimmenden Proteins oder Polypeptids binden und von denen einer markiert oder markierbar ist.

7.  Verfahren nach einem der Ansprüche 4 bis 6, dadurch gekennzeichnet, daß man eine zugesetzte Menge an Thyreoglobulin im Bereich von 0,1 bis 0,5 ng Tg/CT, vorzugsweise von 0,3 ng Tg/CT, in lyophilisierter Form in einem Teströhrchen für ein Meßlösungs-Volumen von 250 µl vorlegt.

8. Kit zur Bestimmung eines Protein oder Polypeptids nach einem der Ansprüche 1 bis 6, dadurch gekennzeichnet, daß er neben den üblichen Kitkomponenten Tracerlösung, Standardlösungen, Nullstandard und Waschflüssigkeit ein Teströhrchen aufweist, das neben einem immobilisierten immunologischen Bindungspartner für das zu bestimmende Protein oder Polypeptid eine feste Menge des zu bestimmenden Proteins oder Polypeptids enthält.

9. Kit nach Anspruch 8, dadurch gekennzeichnet, daß die feste Menge des zu bestimmenden Proteins oder Polypeptids in lyophilisierter Form als Wandbeschichtung des Teströhrchens vorliegt.

10. Kit nach einem der Ansprüche 8 oder 9, dadurch gekennzeichnet, daß das zu bestimmende Protein humanes Thyreoglobulin ist.

11. Teströhrchen zur immunometrischen Bestimmung von Thyreoglobulin in einer Probe einer biologischen Flüssigkeit nach einem Bestimmungsverfahren, bei dem Teströhrchen verwendet werden, an deren Wände Antikörper für die Bindung des zu bestimmenden Thyreoglobulins in der Probe immobilisiert sind, dadurch gekennzeichnet, daß die Wände der Teströhrchen zusätzlich mit einer vorgegebenen Menge an Thyreoglobulin beschichtet sind.

12. Teströhrchen nach Anspruch 11, dadurch gekennzeichnet, daß die vorgegebene Menge an Thyreoglobulin als Beschichtung in einer wasserlöslichen Matrix aufgebracht ist.

13. Teströhrchen nach Anspruch 11 oder 12, dadurch gekennzeichnet, daß die Menge an Thyreoglobulin pro Teströhrchen (CT) im Bereich von 0,1 bis 7 ng/CT liegt.

**Claims**

1. Method for the immunological determination of proteins or polypeptides which are suitable to be used as tumour markers in a sample of a biological fluid by their reaction with immunological binding partners for the protein or polypeptide to be determined, in a measuring solution, and obtaining a measured result which is evaluated using standard curves which are prepared with the aid of standard samples and of a zero standard, further components of proteinic or peptidic nature, which lead to a systematic falsification of the measured values, being likely to be present in the biological fluid, <u>characterized in that</u> both the determination of the proteins or polypeptides in the sample as well as the preparation of the standard curves with the aid of the standard samples and of the zero standard are carried out in the presence of a fixed amount of the protein or polypeptide to be determined, which is added to the measuring solution from the outset, and that the evaluation is carried out in such a way that a measured value which is obtained for a sample and is above the measured value obtained for the zero standard is regarded as a positive measured value which indicates the presence and concentration of the protein or polypeptide to be determined, whereas a measured value which is obtained for a sample and is below the measured value for the zero standard is regarded as an indication of a systematic falsification of the measured value.

2. Method according to Claim 1, characterized in that the determination of the samples and the reactions for the preparation of the standard curves are carried out in a sample vessel which contained the added amount of the protein or polypeptide as a coat on the wall.

3. Method according to Claim 2, characterized in that the test vessel is a coated tube (CT) or a coated microtitre plate which furthermore contains an immobilized immunological binding partner for the protein or polypeptide to be determined.

4. Method according to Claim 1, 2 or 3, characterized in that the protein to be determined is thyroglobulin (Tg).

5. Method according to any of Claims 1 to 4, characterized in that the added amount of protein or polypeptide is chosen so that, in the measurement, it is in the range of 5 to 10 times the amount for the lower detection limit for the protein or polypeptide to be determined in the respective specific immunological assay method.

6. Method according to Claim 4 or 5, characterized in that the assay method is an immunometric assay method known per se, which utilizes two monoclonal antibodies which are used in excess and which bind to different epitopes of the protein or polypeptide to be determined, one of which is labelled or can be labelled.

7. Method according to any of Claims 4 to 6, characterized in that an added amount of thyroglobulin in the range of 0.1 to 0.5 ng Tg/CT, preferably of 0.3 ng Tg/CT, is present in lyophilized form in a test tube for 250 µl of a measuring solution.

8. Kit for the determination of a protein or polypeptide according to any of Claims 1 to 6, characterized in that, in addition to the conventional kit components comprising tracer solution, standard solution, zero standard and washing liquid, it comprises a test tube which, in addition to an immobilized immunological binding partner for the protein or polypeptide to be determined, contains a fixed amount of the protein or polypeptide to be determined.

9. Kit according to Claim 8, characterized in that the fixed amount of the protein or polypeptide to be determined is present in lyophilized form as a coat on the wall of the test tube.

10. Kit according to Claim 8 or 9, characterized in that the protein to be determined is human thyroglobulin.

11. Test tube for the immunometric determination of thyroglobulin in a sample of a biological fluid by a determination method which uses test tubes having immobilized on their walls antibodies for binding the thyroglobulin to be determined in the sample, characterized in that the walls of the test tubes are additionally coated with a predetermined amount of thyroglobulin.

12. Test tube according to Claim 11, characterized in that the predetermined amount of thyroglobulin is applied as a coating in a water-soluble matrix.

13. Test tube according to Claim 11 or 12, characterized in that the amount of thyroglobulin per test tube (CT) is in the range of from 0.1 to 7 ng/CT.

**Revendications**

1. Procédé de détermination immunologique de protéines ou de polypeptides convenant comme marqueurs de tumeurs, dans un échantillon d'un liquide biologique par réaction, dans une solution de mesure, avec des partenaires de liaison immunologique pour la protéine ou le polypeptide à déterminer, et obtention d'un résultat de mesure qui est évalué en recourant à des courbes standard qui sont établies à l'aide d'échantillons standard et d'un témoin, tandis que dans le liquide biologique, il faut tenir compte de la présence d'autres composants de nature protéique ou peptidique qui conduisent à une altération systématique des résultats de mesure, caractérisé en ce que l'on effectue la détermination de la protéine ou du polypeptide dans l'échantillon, ainsi que l'établissement des courbes standard, à l'aide des échantillons standard et du témoin en présence d'une quantité fixe de la protéine ou du polypeptide à déterminer qui est ajoutée préalablement à la solution de mesure, et en ce que l'on effectue l'évaluation en considérant comme valeur de mesure positive indiquant la présence ou la concentration de la protéine ou du polypeptide à déterminer, une valeur de mesure obtenue pour un échantillon et située au-dessus de la valeur de mesure obtenue pour le témoin, tandis que l'on considère une valeur de mesures obtenue pour un échantillon et située en dessous de la valeur de mesure du témoin comme signe d'une altération systématique de la valeur de mesure.

2. Procédé selon la revendication 1, caractérisé en ce que l'on effectue la détermination des échantillons et les réactions d'obtention des courbes standard dans un récipient à échantillon qui contient la quantité ajoutée de la protéine ou du polypeptide sous la forme d'un revêtement préalablement appliqué sur la paroi.

3. Procédé selon la revendication 2, caractérisé en ce que le récipient de test est un tube à essais enduit (CT) ou une plaque de microtitration enduite qui présente en outre un partenaire de liaison immunologique immobilisé pour la protéine ou le polypeptide à déterminer.

4. Procédé selon la revendication 1, 2 ou 3, caractérisé en ce que la protéine à déterminer est la thyréoglobuline (Tg).

5. Procédé selon l'une des revendications 1 à 4, caractérisé en ce que l'on sélectionne la quantité ajoutée de la protéine ou du polypeptide de telle sorte que, lors de la mesure, elle soit située dans la plage de 5 à 10 fois la quantité correspondant à la limite inférieure de détection de la protéine ou du polypeptide à déterminer dans le procédé immunologique de détermination particulier concerné.

6. Procédé selon les revendications 4 ou 5, caractérisé en ce que le procédé de détermination est un procédé de détermination immunométrique connu en soi, qui recourt à deux anticorps monoclonaux utilisés en excès, qui se lient à différents épitopes de la protéine ou du polypeptide à déterminer, et dont l'un est marqué ou peut être marqué.

7. Procédé selon l'une des revendications 4 à 6, caractérisé en ce que, dans un tube à essais prévu pour un volume de solution de mesure de 250 µl, on ajoute préalablement, sous forme lyophilisée, une quantité de thyréoglobuline située dans la plage de 0,1 à 0,5 ng Tg/CT, et de préférence de 0,3 ng Tg/CT.

8. Trousse pour la détermination d'une protéine ou d'un polypeptide selon l'une des revendications 1 à 6, caractérisée en ce qu'en plus des composants habituels de trousse, à savoir une solution de traceur, des solutions standard, un témoin et un liquide de lavage, elle présente un tube à essais qui, en plus d'un partenaire immobilisé, de liaison immunologique pour la protéine ou le polypeptide à déterminer, contient une quantité fixe de la protéine ou du polypeptide à déterminer.

9. Trousse selon la revendication 8, caractérisée en ce que la quantité fixe de la protéine ou du polypeptide à déterminer se présente sous forme lyophilisée comme revêtement de la paroi du tube à essais.

10. Trousse selon l'une des revendications 8 ou 9, caractérisée en ce que la protéine à déterminer est la thyréoglobuline humaine.

11. Tube à essais pour la détermination immunométrique de thyréoglobuline dans un échantillon d'un liquide biologique, selon un procédé de détermination dans lequel on utilise des tubes à essais sur la paroi desquels sont immobilisés des anticorps pour la liaison de la thyréoglobuline à déterminer dans l'échantillon, caractérisé en ce que la paroi des tubes à essais est enduite d'une quantité prédéterminée de thyréoglobuline.

12. Tube à essais selon la revendication 11, caractérisé en ce que la quantité prédéterminée de thyréoglobuline est appliquée sous forme d'un revêtement dans une matrice soluble dans l'eau.

13. Tube à essais selon la revendication 11 ou 12, caractérisé en ce que la quantité de thyréoglobuline par tube à essais (CT) est située dans la plage de 0,1 à 7 ng/CT.

**FIGUR 1**

**FIGUR 2**

**FIGUR 3**